# EUROPEAN PATENT APPLICATION

(11) **EP 2 631 704 A1**
(43) Date of publication of application: **28.08.2013**
(21) Application number: 13156410.6
(22) Date of filing: 22.02.2013
(51) Int. Cl.: G02C 7/04, G02C 7/08, A61F 2/16, H01L 23/58

(54) **Full rings for a functionalized layer insert of an ophthalmic lens**

(30) Priority: 22.02.2012 US 201213402255; 22.02.2012 US 201213402258
(71) Applicant: Johnson & Johnson Vision Care, Inc., Jacksonville, FL 32256 (US)
(72) Inventor: Pugh, Randall B., Jacksonville, FL Florida 32259 (US); Flitsch, Frederick A., New Windsor, NY New York 12553 (US); Otts, Daniel B., Fruit Cove, FL Florida 32259 (US); Riall, James Daniel, St. Johns, FL Florida 32259 (US); Toner, Adam, Jacksonville, FL Florida 32259 (US)
(74) Representative: Tunstall, Christopher Stephen

(57) **Abstract**

An active lens insert for an ophthalmic lens is described. The lens comprising annular shaped full ring substrate layers with one or both of electrical and logical functionality; wherein the size, shape and stacking structure of each of the annular shaped substrate layers is based on the thickness around an optical zone of the ophthalmic lens and electrical interconnections between substrate layers. The active lens insert encapsulated with one or more materials for bonding within the body material of a molded ophthalmic lens. Various designs for rings that make up the functionalized layers in a functional layer insert are described. More specifically, design parameters for the rings for incorporation into an ophthalmic lens. Additionally, functional aspects of the rings and materials for encapsulating the functional insert into an area outside the optical zone of the ophthalmic lens are described.

## Description

The invention relates to an active layer insert for an ophthalmic device formed from multiple functional layers which are stacked and a method for forming the active layer insert. More specifically, various designs for full rings that comprise the functional layers.

### BACKGROUND

Traditionally an ophthalmic device, such as a contact lens, an intraocular lens or a punctal plug included a biocompatible device with a corrective, cosmetic or therapeutic quality. A contact lens, for example, can provide one or more of: vision correcting functionality; cosmetic enhancement; and therapeutic effects. Each function is provided by a physical characteristic of the lens. A design incorporating a refractive quality into a lens can provide a vision corrective function. A pigment incorporated into the lens can provide a cosmetic enhancement. An active agent incorporated into a lens can provide a therapeutic functionality. Such physical characteristics are accomplished without the lens entering into an energized state. A punctal plug has traditionally been a passive device.

More recently, it has been theorized that active components may be incorporated into a contact lens. Some components can include semiconductor devices. Some examples have shown semiconductor devices embedded in a contact lens placed upon animal eyes. It has also been described how the active components may be energized and activated in numerous manners within the lens structure itself. The topology and size of the space defined by the lens structure creates a novel and challenging environment for the definition of various functionalities. Generally, such disclosures have included discrete devices. However, the size and power requirements for available discrete devices are not necessarily conducive for inclusion in a device to be worn on a human eye.

### SUMMARY

According to a first aspect of the invention there is provided an active lens insert for an ophthalmic lens. The lens comprising: annular shaped full ring substrate layers with one or both of electrical and logical Functionality; wherein the size, shape and stacking structure of each of the annular shaped substrate layers is based on the thickness around an optical zone of the ophthalmic lens; electrical interconnections between substrate layers. The active lens insert encapsulated with one or more materials for bonding within the body material of a molded ophthalmic lens.

The substrate functional layers may be adhered to insulating layers forming a stacked feature.

The annular shaped full ring substrate layers may be cut from a wafer.

The size, shape and stacking structure of each of the annular shaped substrate layers may be further based on the base curve of an ophthalmic lens.

The size, shape and stacking structure of each of the annular shaped substrate layers may be further based on by the diameter of an ophthalmic lens.

The size, shape and stacking structure of each of the annular shaped substrate layers is further based on by encapsulation parameters of the active lens insert.

The active lens insert may comprise an encapsulating biocompatible polymer.

The biocompatible polymer for encapsulation may be a polysilicone based polymer.

The encapsulation of the active lens insert may maintains a minimum thickness between an edge of a substrate layer and an outer edge of a lens of less than about 150 micron thickness.

The active lens insert may comprise three or more annular shaped substrate layers.

The substrate insert may comprise a full ring annular shape.

One or more of the substrate layers of the active lens insert may comprise one or more individually functionalized layer.

One or more of the individually functionalized layers may comprise a metallic layer which functions as an antenna.

One or more of the substrate layers of the active lens insert may comprise an energization source.

One or more of the substrate layers of the substrate insert may comprise power regulation source.

The power regulation source may comprise at least one semiconductor layer with electronic microcircuitry capable to control electric current flow from the electrochemical cells.

The electronic microcircuitry may be electrically connected to an electroactive lens component within the ophthalmic lens.

The power regulation of one or more substrate layers may be capable of receiving power from external sources.

The power regulation of one or more substrate layers may be capable of charging the battery layer.

The power regulation of one or more substrate layers may be capable of controlling the use of power when the ophthalmic lens is not in a charging environment.

The power regulation of one or more substrate layers may be capable of controlling the use of power when the ophthalmic lens is in a charging environment.

One or more of the substrate layers of the substrate insert may comprise solid state energy source.

One or more of the substrate layers may comprise microcircuitry to detect actuation signals for the active lens insert.

According to a second aspect of the invention there is provided a method of forming an active lens insert for an ophthalmic lens is described. The method comprising: forming annular shaped full ring substrate layers with one or both of electrical and logical Functionality; wherein the size, shape and stacking structure of each of the annular shaped substrate layers is based on the thickness around an optical zone of the ophthalmic lens; forming electrical interconnections between substrate layers; and encapsulating the active lens insert with one or more materials that may be bonded within the body material of a molded ophthalmic lens.

The method may comprise adhering the substrate functional layers to insulating layers to form a stacked feature.

The annular shaped full ring substrate layers may be cut from a wafer.

The size, shape and stacking structure of each of the annular shaped substrate layers may be further based on the base curve of an ophthalmic lens.

The size, shape and stacking structure of each of the annular shaped substrate layers may be further based on the diameter of an ophthalmic lens.

The size, shape and stacking structure of each of the annular shaped substrate layers may be further based on encapsulation parameters of the active lens insert.

The active lens insert may be encapsulated by a biocompatible polymer.

The biocompatible polymer for encapsulation may be a polysilicone based polymer.

The encapsulation of the active lens insert may maintain a minimum thickness between an edge of a substrate layer and an outer edge of a lens of less than about 150 micron thickness.

The active lens insert may comprise three or more annular shaped substrate layers.

The substrate insert may comprise a full ring annular shape.

One or more of the substrate layers of the active lens insert may comprise one or more individually functionalized layer.

One or more of the individually functionalized layers may comprise a metallic layer which functions as an antenna.

One or more of the substrate layers of the active lens insert may comprise an energization source.

One or more of the substrate layers of the substrate insert may comprise power regulation source.

The power regulation source may comprise at least one semiconductor layer with electronic microcircuitry capable to control electric current flow from the electrochemical cells.

The electronic microcircuitry may be electrically connected to an electroactive lens component within the ophthalmic lens.

The power regulation of one or more substrate layers may be capable of receiving power from external sources.

The power regulation of one or more substrate layers may be capable of charging the battery layer.

The power regulation of one or more substrate layers may be capable of controlling the use of power when the ophthalmic lens is not in a charging environment.

The power regulation of one or more substrate layers may be capable of controlling the use of power when the ophthalmic lens is in a charging environment.

One or more of the substrate layers of the substrate insert may comprise solid state energy source.

One or more of the substrate layers may comprise microcircuitry to detect actuation signals for the active lens insert.

A functionalized layer insert that can be energized and incorporated into an ophthalmic device is described herein. The insert may be formed of multiple layers which may have unique functionality for each layer; or alternatively mixed functionality but in multiple layers. The layers may have layers dedicated to the energization of the product or the activation of the product or for control of functional components within the lens body.

The functionalized layer insert may contain a layer in an energized state which is capable of powering a component capable of drawing a current. Components may include, for example, one or more of: a variable optic lens element, and a semiconductor device, which may either be located in the stacked layer insert or otherwise connected to it. Some examples may also include a cast molded silicone hydrogel contact lens with a rigid or formable insert of stacked functionalized layers contained within the ophthalmic lens in a biocompatible fashion.

Accordingly, there is provided a disclosure of an ophthalmic lens with a stacked functionalized layer portion as well as various designs for rings that comprise the functional layers. Full ring designs parameters can include, for example, thickness, shape, stacking structure, etc. In some examples, design parameters may be influenced by one or more of; the thickness around an optical zone of the lens, the base curve of the lens, the diameter of the lens and encapsulation parameters.

An insert may be formed from multiple layers in various manners and placed in proximity to one, or both of, a first mold part and a second mold part. A reactive monomer mix is placed between the first mold part and the second mold part. The first mold part is positioned proximate to the second mold part thereby forming a lens cavity with the energized substrate insert and at least some of the reactive monomer mix in the lens cavity; the reactive monomer mix is exposed to actinic radiation to form an ophthalmic lens. Lenses may be formed via the control of actinic radiation to which the reactive monomer mixture is exposed.

### DESCRIPTION OF THE DRAWINGS

FIG. 1 illustrates a three dimensional section representation of an insert formed of stacked functional layers which is incorporated within an ophthalmic lens mold part.
FIG. 2 illustrates two cross-sectional representations of inserts formed of stacked functional layers incorporated within two different shaped ophthalmic lenses.
FIG. 3 illustrates two cross-sectional representations of inserts formed of stacked functional layers incorporated within ophthalmic lenses with different encapsulation parameters.
FIG. 4 illustrates two cross-sectional representations of inserts formed of stacked functional layers with different layer thicknesses incorporated within ophthalmic lenses.
FIG. 5A illustrates a top-down view of a wafer with an arrangement of full annular die.
FIG. 5B illustrates a top-down close up view of one full annular die with center cutout.

### DETAILED DESCRIPTION

The present invention relates to an active lens insert device formed through the stacking of multiple functionalized layers. Additionally various designs for a wafer including rings that may be used to make up functionalized layers in a functional layer insert, for incorporation into an ophthalmic lens are provided.

In the following sections detailed descriptions of embodiments of the present invention will be given. The description provides embodiments only, and it is understood that to those skilled in the art that variations, modifications and alterations may be apparent. It is therefore to be understood that said embodiments do not limit the scope of the underlying invention.

### Glossary

In this description and claims directed to the presented invention, various terms may be used for which the following definitions will apply:

Active Lens Insert: as used herein refers to an electronic or electromechanical device with controls based upon logic circuits.

Arc-matched (or arc matching): as used herein refers to the design of a Ring Segment which includes an identical External Radius and Internal Radius, such that the curvature of the External Arc matches the curvature of the Internal Arc. Arc matching is used to efficiently nest Ring Segments on a Wafer, maximizing wafer utilization.

Dicing Street Width: as used herein refers to the width of a thin non-functional space between integrated circuits on a Wafer, where a saw or other device or method can safely cut the Wafer into individual Die without damaging the circuits.

Die: as used herein refers to a block of semiconducting material, on which a given functional circuit is fabricated. Die are created on and cut from a Wafer.

Energized: as used herein refers to the state of being able to supply electrical current to or to have electrical energy stored within.

Energy: as used herein refers to the capacity of a physical system to do work. Many uses within herein may relate to the said capacity being able to perform electrical actions in doing work.

Energy Source: as used herein refers to device capable of supplying Energy or placing a biomedical device in an Energized state.

External Arc: as used herein refers to the external or convex edge of a Ring Segment, which is a portion of the circumference of the circle defined by the External Radius.

External Radius: as used herein refers to the radius of the circle that defines the external edge of a Full Ring or Ring Segment. The External Radius determines the curvature of the External Arc.

Full Ring: as used herein refers to one complete ring-shaped layer in a Functionalized Layer Insert. A Full Ring may be comprised of multiple Ring Segments or may be one Intact Ring.

Functionalized: as used herein refers to making a layer or device able to perform a function including for example, energization, activation, or control.

Functionalized Layer Insert: as used herein refers to an insert for an ophthalmic device formed from multiple functional layers which are stacked. The multiple layers may have unique functionality for each layer; or alternatively mixed functionality but in multiple layers. In some examples, the layers are rings.

Intact Ring: as used herein refers to one complete ring-shaped layer in a Functionalized Layer Insert which is made of a single intact Die.

Internal Arc: as used herein refers to the internal or concave edge of a Ring Segment. The Internal Arc may, be a single arc segment, the curvature of which is determined by the Internal Radius. The Internal Arc may be comprised of multiple arc segments of different curvatures, defined by different Internal Radii.

Internal Radius: as used herein refers to the radius of the circle that defines the internal edge or a portion of the internal edge of a Full Ring or Ring Segment. The Internal Radius determines the curvature of the Internal Arc.

Lens: refers to any ophthalmic device that resides in or on the eye. These devices can provide optical correction or may be cosmetic. For example, the term lens can refer to a contact lens, intraocular lens, overlay lens, ocular insert, optical insert or other similar device through which vision is corrected or modified, or through which eye physiology is cosmetically enhanced (e.g. iris color) without impeding vision. The preferred lenses are soft contact lenses made from silicone elastomers or hydrogels, which include but are not limited to silicone hydrogels, and fluorohydrogels.

Mold: refers to a rigid or semi-rigid object that may be used to form lenses from uncured formulations. Some preferred molds include two mold parts forming a front curve mold part and a back curve mold part.

Power: as used herein refers to work done or energy transferred per unit of time.

Ring Segment: as used herein refers to one Die which may be combined with other Die to construct a Full Ring. As used in this description, a Ring Segment is generally flat and is formed in an arcuate shape.

Stacked: as used herein means to place at least two component layers in proximity to each other such that at least a portion of one surface of one of the layers contacts a first surface of a second layer. A film, whether for adhesion or other functions may reside between the two layers that are in contact with each other through said film.

Substrate insert: as used herein refers to a formable or rigid substrate capable of supporting an Energy Source within an ophthalmic lens. The Substrate insert may also support one or more components.

Wafer: as used herein refers to a thin slice of semiconductor material, such as silicon crystal, used in the fabrication of integrated circuits and other microdevices. The wafer serves as the substrate for microelectronic devices built in and over the wafer and undergoes many microfabrication process steps.

### Apparatus

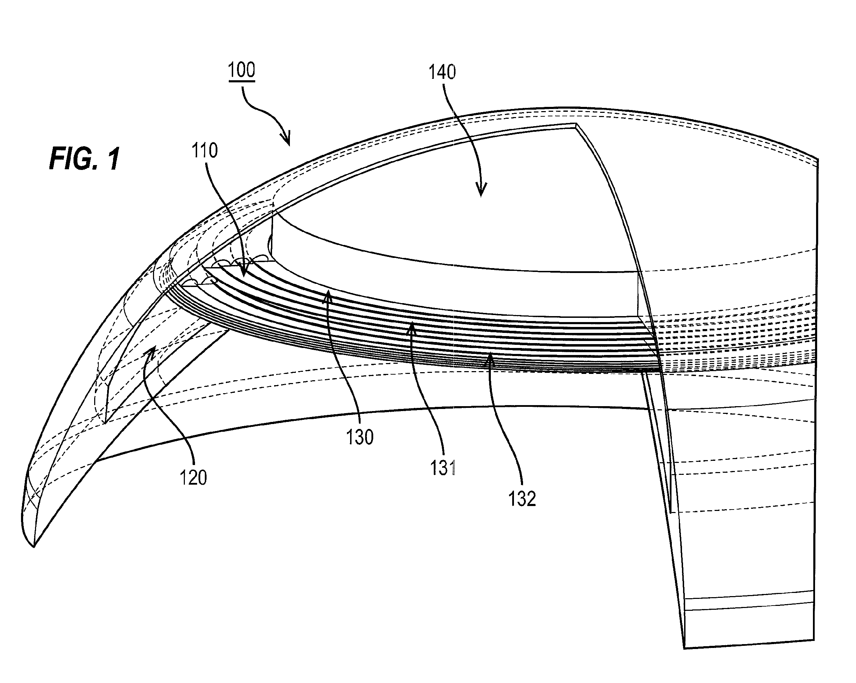

Referring now to Fig. 1, demonstrated as item 100 is a three dimensional representation of an embodiment of the present invention of a fully formed ophthalmic lens using a stacked layer substrate insert formed as a functionalized layer insert 110. The representation shows a partial cut out from the ophthalmic lens to realize the different layers present inside the device. A body material 120 is shown in cross section of the encapsulating layers of the substrate insert. The body material 120 is contained fully within and extends around the entire circumference of the ophthalmic lens. It may be clear to one skilled in the arts that the actual functionalize layer insert 110 may comprise a full annular ring or other shapes that still may reside within the constraints of the size of a typical ophthalmic lens.

Layers 130, 131 and 132 illustrate three of numerous layers that may be found in a functionalized layer insert 110. A single layer may include one or more of: active and passive components and portions with structural, electrical or physical properties conducive to a particular purpose.

A layer 130 may optionally include an energization source, such as, for example, one or more of: a battery, a capacitor and a receiver within the layer 130. Item 131 then, in a non limiting exemplary sense, may comprise microcircuitry in a layer that detects actuation signals for an active lens insert 140. A power regulation layer 132, may be included that is capable of receiving power from external sources, charging the battery layer 130 and controlling the use of battery power from layer 130 when the lens is not in a charging environment. The power regulation layer 132 may also control signals to an exemplary active lens insert 140 in the center annular cutout of the functionalized layer insert 110.

In general, a functionalized layer insert 110 is embodied within an ophthalmic lens via automation which places an energy source a desired location relative to a mold part used to fashion the lens.

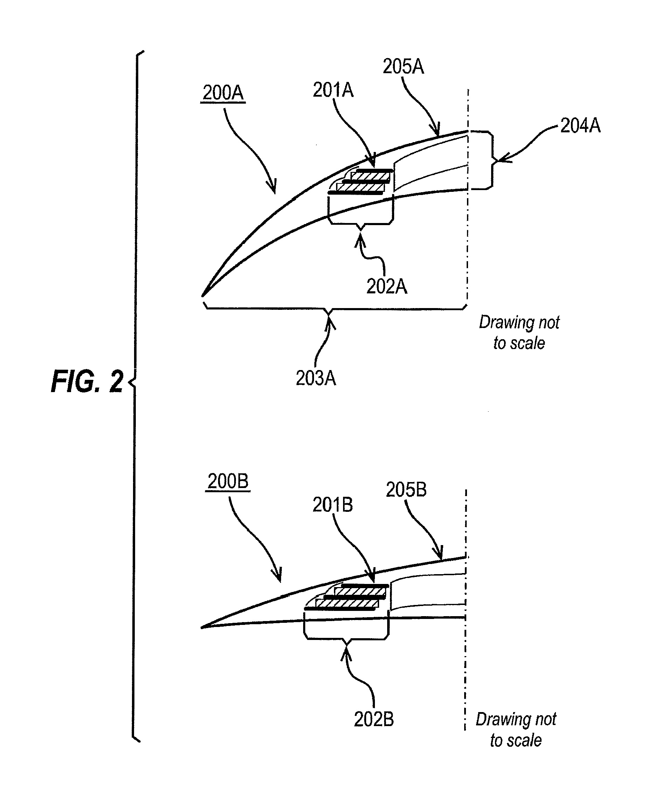

The size, shape, and stacking structure of the die that may be used to form layers such as 130, 131 and 132 in a functionalized layer insert 110 is influenced by several factors, as shown in Figs. 2, 3 and 4.

Fig. 2 illustrates the effect of lens shape on the design of a functionalized layer insert. The base curve, diameter, and thickness of an ophthalmic lens define a maximum size and shape of an included functionalized layer insert. Fig. 2 shows, as one example, the impact of different base curves. Item 200A depicts a cross sectional view of a portion of an ophthalmic lens 205A with more curvature than the ophthalmic lens 205B, depicted in item 200B, which is flatter. The flatter lens 205B can accommodate a functionalized layer insert 201B of greater width 202B, as compared to the narrower width 202A of a functionalized layer insert 201A that fits within lens 205A having greater base curvature. It should be apparent that a lens of smaller diameter (203A indicates a lens diameter) would limit the width of a functionalized layer insert while a lens with larger diameter would accommodate a wider functionalized layer insert. Likewise, a lens of less thickness (204A indicates a lens thickness) would limit the number of layers in a functionalized layer insert as well as the width of a functionalized layer insert, while a thicker lens might support more layers and layers of greater width.

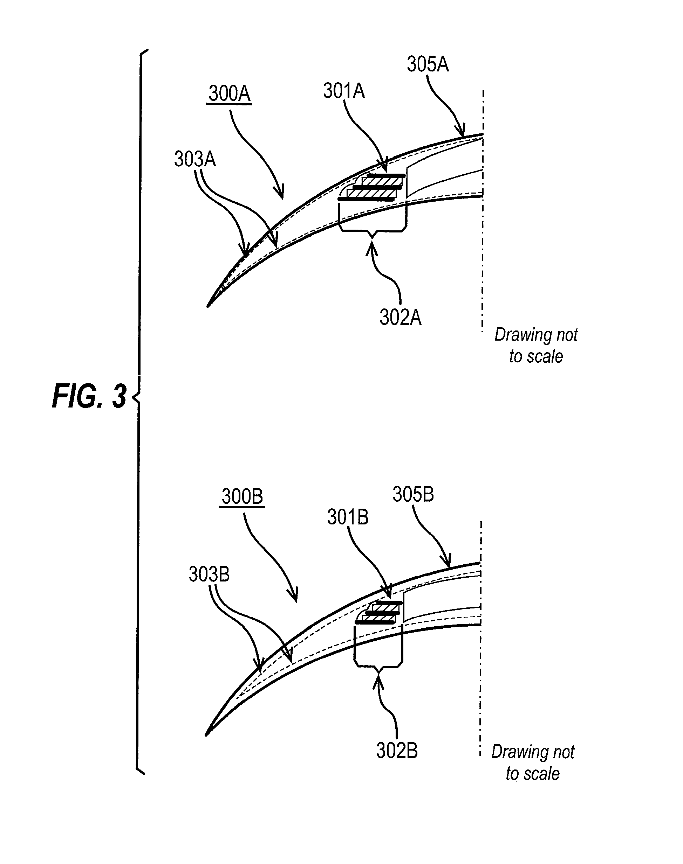

Fig. 3 illustrates the effect of encapsulation parameters on the design of a functionalized layer insert. Encapsulation parameters, such as, by way of non-limiting example, maintaining a minimum 100 micron thickness between the edge of a die and the outer edge of a lens, affect the size and shape of a functionalized layer insert and therefore the size and shape of individual layers. Item 300A depicts a cross-sectional view of a portion of an ophthalmic lens 305A with a functionalized layer insert 301A and encapsulation boundary 303A. The ophthalmic lens 305B depicted in item 300B includes a functionalized layer insert 301B and a relatively wider encapsulation boundary 303B as compared to boundary 303A which is narrower. It can be seen that the wider encapsulation boundary 303B necessitates that the functionalized layer insert 301B be narrower in width 302B as compared to the functionalized layer insert 301A with width 302A.

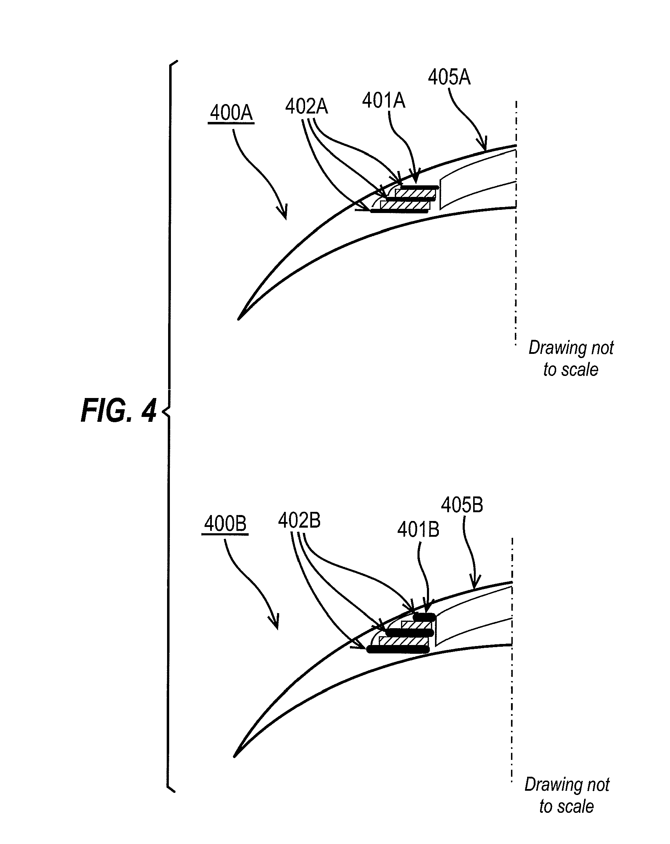

Depicted in Fig. 4 is the effect of functional layer thickness on the design of a functionalized layer insert. Item 400A represents a cross-sectional view of a portion of an ophthalmic lens 405A with a functionalized layer insert 401A including three layers with material, such as, for example, insulating layers, between the functional layers. A functionalized layer insert may contain more or less than three layers. The ophthalmic lens 405B depicted in item 400B includes a functionalized layer insert 401B with relatively thicker layers 402B as compared to the layers 402A in the functionalized layer insert 401A which are thinner. The lens curvature in these two examples allows the width of the bottom layers 402A and 402B to remain the same. However, it can be seen that the increased height of the functionalized layer insert 401B as compared to 401A, combined with the lens curvature, causes the top layer 402A to be limited in width. The thickness of each functional layer impacts other dimensions, such as functional layer width, that will fit within the required lens and encapsulation parameters. Thicker layers within the functionalized layer insert will be more restricted in other dimensions, such as width, in order to remain within the confines of the lens geometry.

The example depicted includes a functionalized layer insert in the shape of a ring, formed as an intact ring-shaped die.

### Full Ring Layout

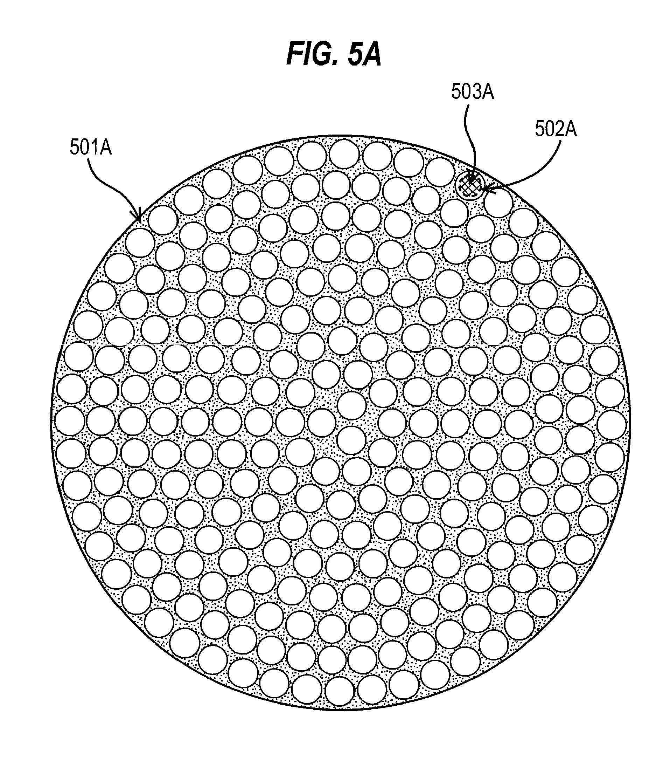

Referring now to Fig. 5A, depicted is a top-down view of an 8-inch wafer 501A with a layout including full ring die 502A with center cutout 503A. The figure shows the area required for each full ring die 502A, but only illustrates an example of the center cutout 503A for one full ring die 502A. Full ring die 502A are positioned adjacent to one another, with at least a dicing street width separation between rings. The most efficient layout includes full ring die 502A arranged in concentric circles around the circumference of the wafer. In this design, significant areas between the individual full ring die 502A are not usable, as well as the center cutout 503A portion of each ring. A layout including full ring die 501 A results in inefficient utilization of a wafer, producing 255 full rings and utilizing only 25.9% of the wafer material.

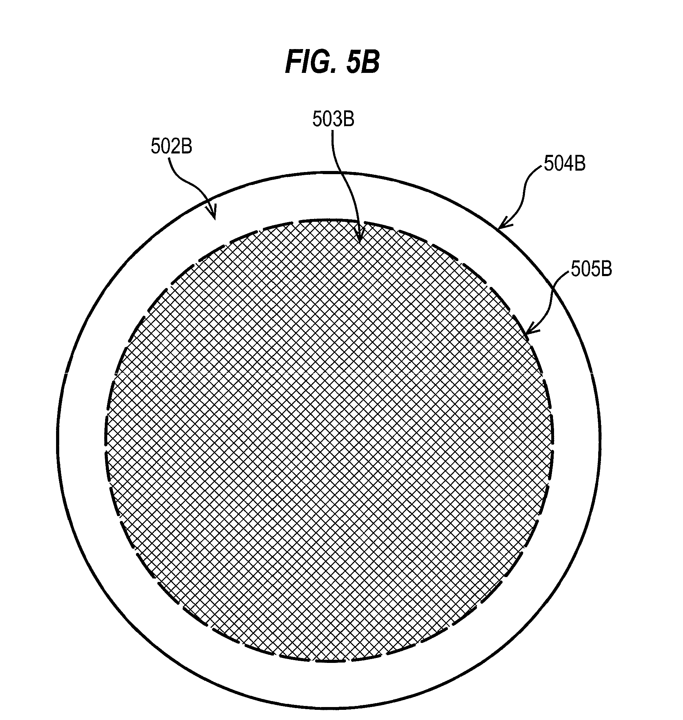

Referring now to Fig. 5B, a top-down close up view of a full ring die 502B is depicted with center cutout 503B. When the center cutout 503B is removed, the full ring die 502B is defined by an outer perimeter 504B and an inner perimeter 505B. The center cutout 503B is unusable after removal from each full ring die 502B produced on a wafer, and is therefore wasted material.

### Conclusion

The description above and as further defined by the claims below, provides various designs for rings that make up the functionalized layers in a functional layer insert, for incorporation into an ophthalmic lens.

## Claims

1. An active lens insert for an ophthalmic lens comprising:
annular shaped full ring substrate layers with one or both of electrical and logical functionality; wherein the size, shape and stacking structure of each of the annular shaped substrate layers is based on the thickness around an optical zone of the ophthalmic lens;
electrical interconnections between substrate layers; and
the active lens insert encapsulated with one or more materials for bonding within the body material of a molded ophthalmic lens.

2. The active lens insert of Claim 1, wherein the substrate functional layers are adhered to insulating layers forming a stacked feature.

3. The active lens insert of Claim 1 or 2, wherein the annular shaped full ring substrate layers are cut from a wafer.

4. The active lens insert of Claim 1, 2 or 3, wherein the size, shape and stacking structure of each of the annular shaped substrate layers is further based on the base curve of an ophthalmic lens.

5. The active lens insert of any preceding claim, wherein the size, shape and stacking structure of each of the annular shaped substrate layers is further based on the diameter of an ophthalmic lens.

6. The active lens insert of any preceding claim, wherein the size, shape and stacking structure of each of the annular shaped substrate layers is further based on encapsulation parameters of the active lens insert.

7. The active lens insert of any preceding claim, further comprising an encapsulating biocompatible polymer.

8. The active lens insert of Claim 7, wherein the biocompatible polymer for encapsulation is a polysilicone based polymer.

9. The active lens insert of any preceding claim, wherein the encapsulation of the active lens insert maintains a minimum thickness between an edge of a substrate layer and an outer edge of a lens of less than about 150 µm thickness.

10. The active lens insert of any preceding claim, wherein the active lens insert comprises three or more annular shaped substrate layers.

11. The active lens insert of any preceding claim, wherein the active lens insert comprises a full ring annular shape.

12. The active lens insert of any preceding claim, wherein one or more of the substrate layers of the active lens insert comprises one or more individually functionalized layer.

13. The active lens insert of Claim 12, wherein one or more of the individually functionalized layers comprises a metallic layer which functions as an antenna.

14. The active lens insert of Claim 10, wherein one or more of the substrate layers of the active lens insert comprises an energization source.

15. The active lens insert of Claim 10 or 14, wherein one or more of the substrate layers of the active lens insert comprises a power regulation source.

16. The active lens insert of Claim 15, wherein the power regulation source comprises at least one semiconductor layer with electronic microcircuitry capable to control electric current flow from the electrochemical cells.

17. The active lens insert of Claim 16, wherein the electronic microcircuitry is electrically connected to an electroactive lens component within the ophthalmic lens.

18. The active lens insert of Claim 15, 16 or 17, wherein the power regulation source of one or more substrate layers are capable of receiving power from external sources.

19. The active lens insert of any one of Claims 15 to 18, wherein the power regulation source of one or more substrate layers are capable of charging the battery layer.

20. The active lens insert of any one of Claims 15 to 19, wherein the power regulation source of one or more substrate layers are capable of controlling the use of power when the ophthalmic lens is not in a charging environment.

21. The active lens insert of any one of Claims 15 to 20, wherein the power regulation source of one or more substrate layers are capable of controlling the use of power when the ophthalmic lens is in a charging environment.

22. The active lens insert of any of Claims 10 to 21, wherein one or more of the substrate layers of the active lens insert comprises a solid state energy source.

23. The active lens insert of any preceding claim, wherein one or more of the substrate layers comprises microcircuitry to detect actuation signals for the active lens insert.

24. A method of forming an active lens insert for an ophthalmic lens, the method comprising:
forming annular shaped full ring substrate layers with one or both of electrical and logical functionality; wherein the size, shape and stacking structure of each of the annular shaped substrate layers is based on the thickness around an optical zone of the ophthalmic lens;
forming electrical interconnections between substrate layers; and
encapsulating the active lens insert with one or more materials for bonding within the body material of a molded ophthalmic lens.
